# EUROPÄISCHE PATENTSCHRIFT

(11) **EP 1 036 078 B1**
(45) Veröffentlichungstag und Bekanntmachung des Hinweises auf die Patenterteilung: **09.05.2007**
(21) Anmeldenummer: 98964411.7
(22) Anmeldetag: 19.11.1998
(51) Int. Cl.: C07D 495/04, A61K 31/505, C07D 333/00, C07D 239/00

(54) **THIENOPYRIMIDINE**
THIENOPYRIMIDINES
THIENOPYRIMIDINES

(30) Priorität: 28.11.1997 DE 19752952
(43) Veröffentlichungstag der Anmeldung: 20.09.2000
(73) Patentinhaber: Merck Patent GmbH, 64293 Darmstadt (DE)
(72) Erfinder: JONAS, Rochus, D-64291 Darmstadt (DE); KLUXEN, Franz-Werner, D-64285 Darmstadt (DE); SCHELLING, Pierre, D-64367 Mühltal (DE); CHRISTADLER, Maria, D-63322 Rödermark (DE)
(86) Internationale Anmeldenummer: PCT/EP1998/007436
(87) Internationale Veröffentlichungsnummer: WO 1999/028325

(56) Entgegenhaltungen:
- EP-A- 0 728 759
- WO-A-94/28902
- WO-A-98/17668
- DD-A- 245 666
- PECH, R. ET AL: "New thieno compounds. Part 12. Preparation of 4-amino substituted thieno[2,3-d]pyrimidin-2-ylacetic acid derivatives" PHARMAZIE (1992), 47(1), 20-1 CODEN: PHARAT;ISSN: 0031-7144, XP002101064

## Beschreibung

Die Erfindung betrifft Verbindungen der Formel I 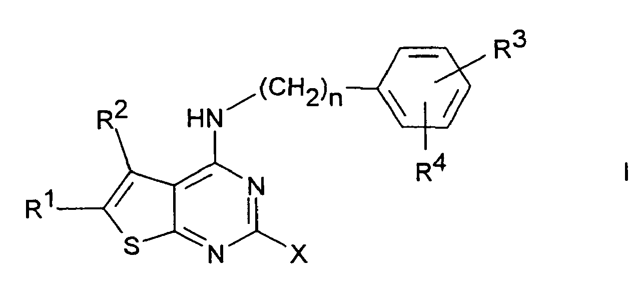 worin
- R¹ und R²: zusammen Alkylen mit 3-5 C-Atomen,
- R³, R⁴: jeweils unabhängig voneinander H, A, OA oder Hal,
- R³ und R⁴: zusammen auch Alkylen mit 3-5 C-Atomen, -O-CH₂-CH₂-, -O-CH₂-O- oder -O-CH₂-CH₂-O-,
- X: einfach durch R⁷ substituiertes R⁵ oder R⁶,
- R⁵: lineares oder verzweigtes Alkylen mit 1-10 C-Atomen,
- R⁶: Cycloalkylalkylen mit 6-12 C-Atomen,
- R⁷: COOH, COOA, CONH₂, CONHA, CON(A)₂ oder CN,
- A: Alkyl mit 1 bis 6 C-Atomen,
- Hal: F, Cl, Br oder I,
- m: 1 oder 2,
und
- n: 1, 2 oder 3
bedeuten,
sowie deren physiologisch unbedenklichen Salze.

Pyrimidinderivate sind beispielsweise aus der EP 201 188 oder der WO 93/06104 bekannt.

In der DD 245 666 A1 werden andere 4-aminosubstituierte Thieno[2,3-d]-pyrimidin-2-yl-essigsäureester beschrieben.

R. Pech et al. beschreiben in Pharmazie (1992), 47(1), 20-21, die Darstellung anderer 4-aminosubstituierter Thieno[2,3-d]-pyrimidin-2-yl-essigsäurederivaten.

Andere Thienopyrimidinderivate werden in EP 0 728 759 A1 als cGMP-PDE-Inhibitoren offenbart.

Wiederum andere Thienopyrimidine mit PDE-V inhibierender Wirkung sind in WO 98 17668 A beschrieben.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.

Insbesondere zeigen sie eine spezifische Inhibierung der cGMP-Phosphodiesterase (PDE V).

Chinazoline mit cGMP-Phosphodiesterase hemmender Aktivität sind z.B. in J. Med. Chem. 36, 3765 (1993) und ibid. 37, 2106 (1994) beschrieben. Die biologische Aktivität der Verbindungen der Formel I kann nach Methoden bestimmt werden, wie sie z.B in der WO 93/06104 beschrieben sind. Die Affinität der erfindungsgemäßen Verbindungen für cGMP- und cAMP-Phosphodiesterase wird durch die Ermittlung ihrer IC₅₀-Werte (Konzentration des Inhibitors, die benötigt wird, um eine 50 %ige Inhibierung der Enzymaktivität zu erreichen) bestimmt.

Zur Durchführung der Bestimmungen können nach bekannten Methoden isolierte Enzyme verwendet werden (z.B. W.J. Thompson et al., Biochem. 1971, 10, 311). Zur Durchführung der Versuche kann eine modifizierte "batch"-Methode von W.J. Thompson und M.M. Appleman (Biochem. 1979, 18, 5228) angewendet werden.

Die Verbindungen eignen sich daher zur Behandlung von Erkrankungen des Herz-Kreislaufsystems, insbesondere der Herzinsuffizienz und zur Behandlung und/oder Therapie von Potenzstörungen (erektile Dysfunktion).

Die Verwendung von substituierten Pyrazolopyrimidinonen zur Behandlung von Impotenz ist z.B. in der WO 94/28902 beschrieben.

Die Verbindungen sind wirksam als Inhibitoren der Phenylepflrin-induzierten Kontraktionen in Corpus cavernosum-Präparationen von Hasen.

Diese biologische Wirkung kann z.B. nach der Methode nachgewiesen werden, die von F. Holmquist et al. in J. Urol., 150, 1310-1315 (1993) beschrieben wird.

Die Inhibierung der Kontraktion, zeigt die Wirksamkeit der erfindungsgemäßen Verbindungen zur Therapie und/oder Behandlung von Potenzstörungen.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zur Herstellung von Verbindungen der Formel I nach Anspruch 1 sowie deren Salzen, dadurch gekennzeichnet, daß man
a) eine Verbindung der Formel II 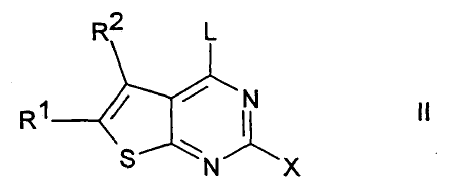 worin
   R¹, R² und X die angegebenen Bedeutungen haben,
   und L Cl, Br, OH, SCH₃ oder eine reaktionsfähige veresterte OH-Gruppe bedeutet,
   mit einer Verbindung der Formel III 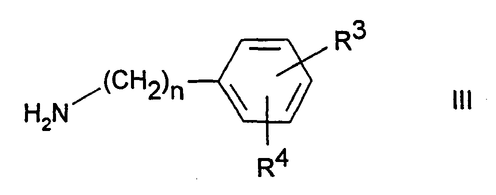 worin
   R³, R⁴ und n die angegebenen Bedeutungen haben,
   umsetzt,
   oder
b) in einer Verbindung der Formel einen Rest X in einen anderen Rest X umwandelt, indem man z.B. eine Estergruppe zu einer COOH-Gruppe hydrolysiert oder eine COOH-Gruppe in ein Amid oder in eine Cyangruppe umwandelt
und/oder daß man eine Verbindung der Formel I in eines ihrer Salze überführt.

Vor- und nachstehend haben die Reste R¹, R², R³, R⁴, R⁵, R⁶, R⁷, X, L und n die bei den Formeln I, II und III angegebenen Bedeutungen, sofern nicht ausdrücklich etwas anderes angegeben ist.

A bedeutet Alkyl mit 1-6 C-Atomen.
In den vorstehenden Formeln ist Alkyl vorzugsweise unverzweigt und hat 1, 2, 3, 4, 5 oder 6 C-Atome und bedeutet vorzugsweise Methyl, Ethyl oder Propyl, weiterhin bevorzugt Isopropyl, Butyl, Isobutyl, sek.-Butyl oder tert.-Butyl, aber auch n-Pentyl, Neopentyl, Isopentyl oder Hexyl.

X bedeutet einen einfach durch R⁷ substituierten R⁵ oder R⁶-Rest.

R⁵ bedeutet einen linearen oder verzweigten Alkylenrest mit 1-10, vorzugsweise 1-8 C-Atomen, wobei der Alkylenrest vorzugsweise z.B. Methylen, Ethylen, Propylen, Isopropylen, Butylen, Isobutylen, sek.-Butylen, Pentylen, 1-, 2- oder 3-Methylbutylen, 1,1- , 1,2- oder 2,2-Dimethylpropylen, 1-Ethylpropylen, Hexylen, 1- , 2- , 3- oder 4-Methylpentylen, 1,1-, 1,2- , 1,3- , 2,2- , 2,3- oder 3,3-Dimethylbutylen, 1- oder 2-Ethylbutylen, 1-Ethyl-1-methylpropylen, 1-Ethyl-2-methylpropylen, 1,1,2- oder 1,2,2-Trimethylpropylen, lineares oder verzweigtes Heptylen, Octylen, Nonylen oder Decylen bedeutet.

R⁶ bedeutet Cycloalkylalkylen mit 6-12 C-Atomen, vorzugsweise z.B. Cycclopentylmethylen, Cyclohexylmethylen, Cyclohexylethylen, Cyclohexylpropylen oder Cyclohexylbutylen.

Ferner bedeuten R¹ und R² zusammen bevorzugt Propylen, Butylen oder Pentylen.

Hal bedeutet vorzugsweise F, Cl oder Br, aber auch I.

Die Reste R³ und R⁴ können gleich oder verschieden sein und stehen vorzugsweise in der 3- oder 4-Position des Phenylrings. Sie bedeuten beispielsweise jeweils unabhängig voneinander H, Alkyl, F, Cl, Br oder I oder zusammen Alkylen, wie z.B. Propylen, Butylen oder Pentylen, ferner Ethylenoxy, Methylendioxy oder Ethylendioxy. Bevorzugt stehen sie auch jeweils für Alkoxy, wie z.B. für Methoxy, Ethoxy oder Propoxy.

Der Rest R⁷ bedeutet vorzugsweise z.B. COOH, COOCH₃, COOC₂H₅, CONH₂, CON(CH₃)₂, CONHCH₃ oder CN.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis lb ausgedrückt werden, die der Formel I entsprechen und worin die nicht näher bezeichneten Reste die bei der Formel I angegebene Bedeutung haben, worin jedoch
- in la: X durch COOH oder COOA substituiertes R⁵ oder R⁶bedeuten;
- in lb: R¹ und R² zusammen Alkylen mit 3-5 C-Atomen,
R³, R⁴ jeweils unabhängig voneinander H, A, OA oder Hal,
R³und R⁴ zusammen auch -O-CH₂-O-,
X einfach durch R⁷ substituiertes R⁵,
R⁵ lineares oder verzweigtes Alkylen mit 1-10 C-Atomen,
R⁷ COOH oder COOA,
A Alkyl mit 1 bis 6 C-Atomen,
Hal F, Cl, Br oder I,
m 1 und
n 1 oder 2 bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II oder III haben R¹, R², R³, R⁴, X und n die angegebenen Bedeutungen, insbesondere die angegebenen bevorzugten Bedeutungen.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy, ferner auch 2-Naphthalinsulfonyloxy).

Die Verbindungen der Formel I können vorzugsweise erhalten werden, indem man Verbindungen der Formel II mit Verbindungen der Formel III umsetzt.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Andererseits ist es möglich, die Reaktion stufenweise durchzuführen.

Die Ausgangsverbindungen der Formel II und III sind in der Regel bekannt. Sind sie nicht bekannt, so können sie nach an sich bekannten Methoden hergestellt werden.
Verbindungen der Formel II können z.B. durch Umsetzung mit POCl₃ aus Verbindungen erhalten werden, die aus Thiophenderivaten und CNsubstituierten Alkylencarbonsäureestem aufgebaut werden (Eur. J. Med. Chem. 23, 453 (1988).

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropytether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Es ist ferner möglich, in einer Verbindung der Formel I einen Rest X in einen anderen Rest X umzuwandeln, z.B. indem man einen Ester oder eine Cyangruppe zu einer COOH-Gruppe hydrolysiert. Estergruppen können z.B. mit NaOH oder KOH in Wasser, Wasser-THF oder Wasser-Dioxan bei Temperaturen zwischen 0 und 100° verseift werden.
Carbonsäuren können z.B. mit Thionylchlorid in die entsprechenden Carbonsäurechloride und diese in Carbonsäureamide umgewandelt werden. Durch Wasserabspaltung in bekannter Weise erhält man aus diesen Carbonitrile.

Eine Säure der Formel I kann mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann die Säure der Formel I mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.
Für diese Umsetzung kommen insbesondere auch organische Basen in Frage, die physiologisch unbedenkliche Salze liefern, wie z.B. Ethanolamin.

Andererseits kann eine Base der Formel I mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind auch Arzneimittel der formel I und ihre physiologisch unbedenklichen Salze als Phosphodiesterase V-Hemmer.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cGMP(cyclo-Guanosin-monophosphat)-Spiegels zu Entzündungshemmung oder -verhinderung und Muskelentspannung führt, eingesetzt werden. Besondere Verwendung können die erfindungsgemäßen Verbindungen bei der Behandlung von Krankheiten des Herz-Kreislaufsystems und zur Behandlung und/oder Therapie von Potenzstörungen finden.

Dabei werden die Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

| | |
|---|---|
| Massenspektrometrie (MS): | EI (Elektronenstoß-lonisation) M⁺ |
| | FAB (Fast Atom Bombardment) (M+H)⁺ |

### Beispiel 1

1,9 g 3-(4-Chlor-5,6,7,8-tetrahydro-[1]-benzothieno-[2,3-d]-pyrimidin-2-yl)-propionsäuremethylester [erhältlich durch Cyclisierung von 2-Amino-4,5,6,7-tetrahydrobenzothiophen-3-carbonsäuremethylester mit 3-Cyanpropionsäuremethylester und nachfolgender Chlorierung mit Phosphoroxichlorid/Dimethylamin] und 2,3 g 3-Chlor-4-methoxybenzylamin ("A") in 20 ml N-Methylpyrrolidon werden 5 Stunden bei 110° gerührt. Das Lösungsmittel wird entfernt und wie üblich aufgearbeitet. Man erhält 2,6 g 3-[4-(3-Chlor-4-methoxy-benzylamino)-5,6,7,8-tetrahydro-[1]-benzothieno-[2,3-d]-pyrimidin-2-yl]-propionsäuremethylester als farbloses Öl.

Analog erhält man durch Umsetzung von "A"
mit 3-(4-Chlor-5,6-cyclopenteno-[1]-benzothieno-[2,3-d]-pyrimidin-2-yl)-propionsäuremethylester
   3-[4-(3-Chlor-4-methoxy-benzylamino)-5,6-cyclopenteno-[1]-benzothieno-[2,3-d]-pyrimidin-2-yl]-propionsäuremethylester;
mit 3-(4-Chlor-5,6-cyclohepteno-[1]-benzothieno-[2,3-d]-pyrimidin-2-yl)-propionsäuremethylester
   3-[4-(3-Chlor-4-methoxy-benzylamino)-5,6-cyclohepteno-[1]-benzothieno-[2,3-d]-pyrimidin-2-yl]-propionsäuremethylester;
mit 2-(4-Chlor-5,6,7,8-tetrahydro-[1]-benzothieno-[2,3-d]-pyrimidin-2-yl)-essigsäuremethylester
   2-[4-(3-Chlor-4-methoxy-benzylamino)-5,6,7,8-tetrahydro-[1]-benzothieno-[2,3-d]-pyrimidin-2-yl]-essigsäuremethylester.

Analog erhält man durch Umsetzung von 3,4-Methylendioxybenzylamin
mit 3-(4-Chlor-5,6,7,8-tetrahydro-[1]-benzothieno-[2,3-d]-pyrimidin-2-yl)-propionsäuremethylester
   3-[4-(3,4-Methylendioxy-benzylamino)-5,6,7,8-tetrahydro-[1]-benzothieno-[2,3-d]-pyrimidin-2-yl]-propionsäuremethylester;
mit 3-(4-Chlor-5,6-cyclopenteno-[1]-benzothieno-[2,3-d]-pyrimidin-2-yl)-propionsäuremethylester
   3-[4-(3,4-Methylendioxy-benzylamino)-5,6-cyclopenteno-[1]-benzothieno-[2,3-d]-pyrimidin-2-yl]-propionsäuremethylester;
mit 3-(4-Chlor-5,6-cyclohepteno-[1]-benzothieno-[2,3-d]-pyrimidin-2-yl)-propionsäuremethylester
   3-[4-(3,4-Methylendioxy-benzylamino)-5,6-cyclohepteno-[1]-benzothieno-[2,3-d]-pyrimidin-2-yl]-propionsäuremethylester.

Analog erhält man durch Umsetzung von "A"
mit 4-(4-Chlor-5,6,7,8-tetrahydro-[1]-benzothieno-[2,3-d]-pyrimidin-2-yl)-buttersäuremethylester
   4-[4-(3-Chlor-4-methoxy-benzylamino)-5,6,7,8-tetrahydro-[1]-benzothieno-[2,3-d]-pyrimidin-2-yl]-buttersäuremethylester;
mit 4-(4-Chlor-5,6-cyclopenteno-[1]-benzothieno-[2,3-d]-pyrimidin-2-yl)-buttersäuremethylester
   4-[4-(3-Chlor-4-methoxy-benzylamino)-5,6-cyclopenteno-[1]-benzothieno-[2,3-d]-pyrimidin-2-yl]-buttersäuremethylester;
mit 4-(4-Chlor-5,6-cyclohepteno-[1]-benzothieno-[2,3-d]-pyrimidin-2-yl)-buttersäuremethylester
   4-[4-(3-Chlor-4-methoxy-benzylamino)-5,6-cyclohepteno-[1]-benzothieno-[2,3-d]-pyrimidin-2-yl]-buttersäuremethylester.

Analog erhält man durch Umsetzung von 3,4-Methylendioxybenzylamin
mit 4-(4-Chlor-5,6,7,8-tetrahydro-[1]-benzothieno-[2,3-d]-pyrimidin-2-yl)-buttersäuremethylester
   4-[4-(3,4-Methylendioxy-benzylamino)-5,6,7,8-tetrahydro-[1]-benzothieno-[2,3-d]-pyrimidin-2-yl]-buttersäuremethylester;
mit 4-(4-Chlor-5,6-cyclopenteno-[1]-benzothieno-[2,3-d]-pyrimidin-2-yl)-buttersäuremethylester
   4-[4-(3,4-Methylendioxy-benzylamino)-5,6-cyclopenteno-[1]-benzothieno-[2,3-d]-pyrimidin-2-yl]-buttersäuremethylester;
mit 4-(4-Chlor-5,6-cyclohepteno-[1]-benzothieno-[2,3-d]-pyrimidin-2-yl)-buttersäuremethylester
   4-[4-(3,4-Methylendioxy-benzylamino)-5,6-cyclohepteno-[1]-benzothieno-[2,3-d]-pyrimidin-2-yl]-buttersäuremethylester.

Analog erhält man durch Umsetzung von "A"
mit 5-(4-Chlor-5,6,7,8-tetrahydro-[1]-benzothieno-[2,3-d]-pyrimidin-2-yl)-valeriansäuremethylester
   5-[4-(3-Chlor-4-methoxy-benzylamino)-5,6,7,8-tetrahydro-[1]-benzothieno-[2,3-d]-pyrimidin-2-yl]-valeriansäuremethylester;
mit 5-(4-Chlor-5,6-cyclopenteno-[1]-benzothieno-[2,3-d]-pyrimidin-2-yl)-valeriansäuremethylester
   5-[4-(3-Chlor-4-methoxy-benzylamino)-5,6-cyclopenteno-[1]-benzothieno-[2,3-d]-pyrimidin-2-yl]-valeriansäuremethylester;
mit 5-(4-Chlor-5,6-cyclohepteno-[1]-benzothieno-[2,3-d]-pyrimidin-2-yl)-valeriansäuremethylester
   5-[4-(3-Chlor-4-methoxy-benzylamino)-5,6-cyclohepteno-[1]-benzothieno-[2,3-d]-pyrimidin-2-yl]-valeriansäuremethylester.

Analog erhält man durch Umsetzung von 3,4-Methylendioxybenzylamin
mit 5-(4-Chlor-5,6,7,8-tetrahydro-[1]-benzothieno-[2,3-d]-pyrimidin-2-yl)-valeriansäuremethylester
   5-[4-(3,4-Methylendioxy-benzylamino)-5,6,7,8-tetrahydro-[1]-benzothieno-[2,3-d]-pyrimidin-2-yl]-valeriansäuremethylester;
mit 5-(4-Chlor-5,6-cyclopenteno-[1]-benzothieno-[2,3-d]-pyrimidin-2-yl)-valeriansäuremethylester
   5-[4-(3,4-Methylendioxy-benzylamino)-5,6-cyclopenteno-[1]-benzothieno-[2,3-d]-pyrimidin-2-yl]-valeriansäuremethylester;
mit 5-(4-Chlor-5,6-cyclohepteno-[1]-benzothieno-[2,3-d]-pyrimidin-2-yl)-valeriansäuremethylester
   5-[4-(3,4-Methylendioxy-benzylamino)-5,6-cyclohepteno-[1]-benzothieno-[2,3-d]-pyrimidin-2-yl]-valeriansäuremethylester.

Analog erhält man durch Umsetzung von "A"
mit 7-(4-Chlor-5,6,7,8-tetrahydro-[1]-benzothieno-[2,3-d]-pyrimidin-2-yl)-heptansäuremethylester
   7-[4-(3-Chlor-4-methoxy-benzylamino)-5,6,7,8-tetrahydro-[1]-benzothieno-[2,3-d]-pyrimidin-2-yl]-heptansäuremethylester;
mit 7-(4-Chlor-5,6-cyclopenteno-[1]-benzothieno-[2,3-d]-pyrimidin-2-yl)-heptansäuremethylester
   7-[4-(3-Chlor-4-methoxy-benzylamino)-5,6-cyclopenteno-[1]-benzothieno-[2,3-d]-pyrimidin-2-yl]-heptansäuremethylester;
mit 7-(4-Chlor-5,6-cyclohepteno-[1]-benzothieno-[2,3-d]-pyrimidin-2-yl)-heptansäuremethylester
   7-[4-(3-Chlor-4-methoxy-benzylamino)-5,6-cyclohepteno-[1]-benzothieno-[2,3-d]-pyrimidin-2-yl]-heptansäuremethylester.

Analog erhält man durch Umsetzung von 3,4-Methylendioxybenzylamin
mit 7-(4-Chlor-5,6,7,8-tetrahydro-[1]-benzothieno-[2,3-d]-pyrimidin-2-yl)-heptansäuremethylester
   7-[4-(3,4-Methylendioxy-benzylamino)-5,6,7,8-tetrahydro-[1]-benzothieno-[2,3-d]-pyrimidin-2-yl]-heptansäuremethylester;
mit 7-(4-Chlor-5,6-cyclopenteno-[1]-benzothieno-[2,3-d]-pyrimidin-2-yl)-heptansäuremethylester
   7-[4-(3,4-Methylendioxy-benzylamino)-5,6-cyclopenteno-[1]-benzothieno-[2,3-d]-pyrimidin-2-yl]-heptansäuremethylester;
mit 7-(4-Chlor-5,6-cyclohepteno-[1]-benzothieno-[2,3-d]-pyrimidin-2-yl)-heptansäuremethylester
   7-[4-(3,4-Methylendioxy-benzylamino)-5,6-cyclohepteno-[1]-benzothieno-[2,3-d]-pyrimidin-2-yl]-heptansäuremethylester.

Analog erhält man durch Umsetzung von "A"
mit 2-[4-(4-Chlor-5,6,7,8-tetrahydro-[1]-benzothieno-[2,3-d]-pyrimidin-2-yl)-cyclohexyl-1-yl]-essigsäuremethylester
   2-{4-[4-(3-Chlor-4-methoxy-benzylamino)-5,6,7,8-tetrahydro-[1]-benzothieno-[2,3-d]-pyrimidin-2-yl]-cyclohexyl-1-yl}-essigsäuremethylester.

Analog erhält man durch Umsetzung von 3,4-Methylendioxybenzylamin
mit 2-[4-(4-Chlor-5,6,7,8-tetrahydro-[1]-benzothieno-[2,3-d]-pyrimidin-2-yl)-cyclohexyl-1-yl]-essigsäuremethylester
   2-{4-[4-(3,4-Methylendioxy-benzylamino)-5,6,7,8-tetrahydro-[1]-benzothieno-[2,3-d]-pyrimidin-2-yl]-cyclohexyl-1-yl}-essigsäuremethylester.

Analog erhält man durch Umsetzung von Benzylamin
mit 3-(4-Chlor-5,6,7,8-tetrahydro-[1]-benzothieno-[2,3-d]-pyrimidin-2-yl)-propionsäuremethylester
   3-(4-Benzylamino-5,6,7,8-tetrahydro-[1]-benzothieno-[2,3-d]-pyrimidin-2-yl)-propionsäuremethylester;
mit 4-(4-Chlor-5,6,7,8-tetrahydro-[1]-benzothieno-[2,3-d]-pyrimidin-2-yl)-buttersäuremethylester
   4-(4-Benzylamino-5,6,7,8-tetrahydro-[1]-benzothieno-[2,3-d]-pyrimidin-2-yl)-buttersäuremethylester;
mit 5-(4-Chlor-5,6,7,8-tetrahydro-[1]-benzothieno-[2,3-d]-pyrimidin-2-yl)-valeriansäuremethylester
   5-(4-Benzylamino-5,6,7,8-tetrahydro-[1]-benzothieno-[2,3-d]-pyrimidin-2-yl)-valeriansäuremethylester.

### Beispiel 2

2.2g 3-[4-(3-Chlor-4-methoxy-benzylamino)-5,6,7,8-tetrahydro-[1]-benzothieno-[2,3-d]-pyrimidin-2-yl]-propionsäuremethylester wird in 20 ml Ethylenglycolmonomethylether gelöst und nach Zugabe von 10 ml 32 %iger NaOH 5 Stunden bei 110° gerührt. Nach Zugabe von 20 %iger HCl wird mit Dichlormethan extrahiert. Durch Zugabe von Petrolether erhält man 2,0 g 3-[4-(3-Chlor-4-methoxy-benzylamino)-5,6,7,8-tetrahydro-[1]-benzothieno-[2,3-d]-pyrimidin-2-yl]-propionsäure, F. 229°.
Die ausgefallenen Kristalle werden in 30 ml Isopropanol gelöst und mit 0,5 g Ethanolamin versetzt. Nach Kristallisation erhält man 1,35 g 3-[4-(3-Chlor-4-methoxy-benzylamino)-5,6,7,8-tetrahydro-[1]-benzothieno-[2,3-d]-pyrimidin-2-yl]-propionsäure, Ethanolaminsalz, F. 135°.

Analog erhält man aus den unter Beispiel 1 aufgeführten Estern die nachstehenden Carbonsäuren:
3-[4-(3-Chlor-4-methoxy-benzylamino)-5,6-cyclopenteno-[1]-benzothieno-[2,3-d]-pyrimidin-2-yl]-propionsäure;
3-[4-(3-Chlor-4-methoxy-benzylamino)-5,6-cyclohepteno-[1]-benzothieno-[2,3-d]-pyrimidin-2-yl]-propionsäure;
2-[4-(3-Chlor-4-methoxy-benzylamino)-5,6,7,8-tetrahydro-[1]-benzothieno-[2,3-d]-pyrimidin-2-yl]-essigsäure, Ethanolaminsalz, F. 126°;
3-[4-(3,4-Methylendioxy-benzylamino)-5,6,7,8-tetrahydro-[1]-benzothieno-[2,3-d]-pyrimidin-2-yl]-propionsäure;
3-[4-(3,4-Methylendioxy-benzylamino)-5,6-cyclopenteno-[1]-benzothieno-[2,3-d]-pyrimidin-2-yl]-propionsäure;
3-[4-(3,4-Methylendioxy-benzylamino)-5,6-cyclohepteno-[1]-benzothieno-[2,3-d]-pyrimidin-2-yl]-propionsäure;
4-[4-(3-Chlor-4-methoxy-benzylamino)-5,6,7,8-tetrahydro-[1]-benzothieno-[2,3-d]-pyrimidin-2-yl]-buttersäure;
4-[4-(3-Chlor-4-methoxy-benzylamino)-5,6-cyclopenteno-[1]-benzothieno-[2,3-d]-pyrimidin-2-yl]-buttersäure;
4-[4-(3-Chlor-4-methoxy-benzylamino)-5,6-cyclohepteno-[1]-benzothieno-[2,3-d]-pyrimidin-2-yl]-buttersäure;
4-[4-(3,4-Methylendioxy-benzylamino)-5,6,7,8-tetrahydro-[1]-benzothieno-[2,3-d]-pyrimidin-2-yl]-buttersäure, Ethanolaminsalz, F. 114°;
4-[4-(3,4-Methylendioxy-benzylamino)-5,6-cyclopenteno-[1]-benzothieno-[2,3-d]-pyrimidin-2-yl]-buttersäure;
4-[4-(3,4-Methylendioxy-benzylamino)-5,6-cyclohepteno-[1]-benzothieno-[2,3-d]-pyrimidin-2-yl]-buttersäure;
5-[4-(3-Chlor-4-methoxy-benzylamino)-5,6,7,8-tetrahydro-[1]-benzothieno-[2,3-d]-pyrimidin-2-yl]-valeriansäure, F. 165°; Ethanolaminsalz, F. 112°;
5-[4-(3-Chlor-4-methoxy-benzylamino)-5,6-cyclopenteno-[1]-benzothieno-[2,3-d]-pyrimidin-2-yl]-valeriansäure;
5-[4-(3-Chlor-4-methoxy-benzylamino)-5,6-cyclohepteno-[1]-benzothieno-[2,3-d]-pyrimidin-2-yl]-valeriansäure;
5-[4-(3,4-Methylendioxy-benzylamino)-5,6,7,8-tetrahydro-[1]-benzothieno-[2,3-d]-pyrimidin-2-yl]-valeriansäure;
5-[4-(3,4-Methylendioxy-benzylamino)-5,6-cyclopenteno-[1]-benzothieno-[2,3-d]-pyrimidin-2-yl]-valeriansäure;
5-[4-(3,4-Methylendioxy-benzylamino)-5,6-cyclohepteno-[1]-benzothieno-[2,3-d]-pyrimidin-2-yl]-valeriansäure;
7-[4-(3-Chlor-4-methoxy-benzylamino)-5,6,7,8-tetrahydro-[1]-benzothieno-[2,3-d]-pyrimidin-2-yl]-heptansäure, Ethanolaminsalz, F. 130°;
7-[4-(3-Chlor-4-methoxy-benzylamino)-5,6-cyclopenteno-[1]-benzothieno-[2,3-d]-pyrimidin-2-yl]-heptansäure;
7-[4-(3-Chlor-4-methoxy-benzylamino)-5,6-cyclohepteno-[1]-benzothieno-[2,3-d]-pyrimidin-2-yl]-heptansäure;
7-[4-(3,4-Methylendioxy-benzylamino)-5,6,7,8-tetrahydro-[1]-benzothieno-[2,3-d]-pyrimidin-2-yl]-heptansäure, Ethanolaminsalz, F. 137°;
7-[4-(3,4-Methylendioxy-benzylamino)-5,6-cyclopenteno-[1]-benzothieno-[2,3-d]-pyrimidin-2-yl]-heptansäure;
7-[4-(3,4-Methylendioxy-benzylamino)-5,6-cyclohepteno-[1]-benzothieno-[2,3-d]-pyrimidin-2-yl]-heptansäure;
2-{4-[4-(3-Chlor-4-methoxy-benzylamino)-5,6,7,8-tetrahydro-[1]-benzothieno-[2,3-d]-pyrimidin-2-yl]-cyclohexyl}-essigsäure;
2-{4-[4-(3,4-Methylendioxy-benzylamino)-5,6,7,8-tetrahydro-[1]-benzothieno-[2,3-d]-pyrimidin-2-yl]-cyclohexyl}-essigsäure;
3-(4-Benzylamino-5,6,7,8-tetrahydro-[1]-benzothieno-[2,3-d]-pyrimidin-2-yl)-propionsäure, Ethanolaminsalz, F. 126°;
4-(4-Benzylamino-5,6,7,8-tetrahydro-[1]-benzothieno-[2,3-d]-pyrimidin-2-yl)-buttersäure, Ethanolaminsalz, F. 133°;
5-(4-Benzylamino-5,6,7,8-tetrahydro-[1]-benzothieno-[2,3-d]-pyrimidin-2-yl)-valeriansäure, Ethanolaminsalz, F. 135°.

### Beispiel 3

1 Äquivalent 3-[4-(3-Chlor-4-methoxy-benzylamino)-5,6,7,8-tetrahydro-[1]-benzothieno-[2,3-d]-pyrimidin-2-yl]-propionsäure und 1,2 Äquivalente Thionylchlorid werden 2 Stunden in Dichlormethan gerührt. Das Lösungsmittel wird entfernt und man erhält 3-[4-(3-Chlor-4-methoxy-benzylamino)-5,6,7,8-tetrahydro-[1]-benzothieno-[2,3-d]-pyrimidin-2-yl]-propionsäurechlorid.
Man überführt in wässriges Ammoniak, rührt eine Stunde und erhält nach üblicher Aufarbeitung 3-[4-(3-Chlor-4-methoxy-benzylamino)-5,6,7,8-tetrahydro-[1]-benzothieno-[2,3-d]-pyrimidin-2-yl]-propionsäureamid.

### Beispiel 4

1 Äquivalent DMF und 1 Äquivalent Oxalylchlorid werden bei 0° in Acetonitril gelöst. Danach wird 1 Äquivalent 3-[4-(3-Chlor-4-methoxy-benzylamino)-5,6,7,8-tetrahydro-[1]-benzothieno-[2,3-d]-pyrimidin-2-yl]-propionsäureamid zugegeben. Es wird eine Stunde nachgerührt. Nach üblicher Aufarbeitung erhält man 3-[4-(3-Chlor-4-methoxy-benzylamino)-5,6,7,8-tetrahydro-[1]-benzothieno-[2,3-d]-pyrimidin-2-yl]-propionitril.

### Beispiel 5

Analog zu den Beispielen 1 und 2 werden die nachstehenden Verbindungen erhalten
6-[4-(3-Chlor-4-methoxy-benzylamino)-5,6,7,8-tetrahydro-[1]-benzothieno-[2,3-d]-pyrimidin-2-yl]-hexansäure, F. 165°;
2-[4-(3-Chlor-4-methoxy-benzylamino)-5,6,7,8-tetrahydro-[1]-benzothieno-[2,3-d]-pyrimidin-2-yl]-propionsäure, Ethanolaminsalz, F. 150°;
4-[4-(3-Chlor-4-methoxy-benzylamino)-5,6,7,8-tetrahydro-[1]-benzothieno-[2,3-d]-pyrimidin-2-yl]-2,2-dimethyl-buttersäure, Ethanolaminsalz, F. 130°;
4-[4-(3,4-Methylendioxy-benzylamino)-5,6,7,8-tetrahydro-[1]-benzothieno-[2,3-d]-pyrimidin-2-yl]-2,2-dimethyl-buttersäure, Ethanolaminsalz, F. 126°;
5-[4-(3-Chlor-4-hydroxy-benzylamino)-5,6,7,8-tetrahydro-[1]-benzothieno-[2,3-d]-pyrimidin-2-yl]-valeriansäure, F. 179°;
5-[4-(3,4-Dichlor-benzylamino)-5,6,7,8-tetrahydro-[1]-benzothieno-[2,3-d]-pyrimidin-2-yl]-valeriansäure, Ethanolaminsalz F. 136°;
5-[4-(3-Chlor-4-isopropyloxy-benzylamino)-5,6,7,8-tetrahydro-[1]-benzothieno-[2,3-d]-pyrimidin-2-yl]-valeriansäure, Ethanolaminsalz, F. 118°;
2-[4-(4-(3-Chlor-4-methoxy-benzylamino)-5,6,7,8-tetrahydro-[1]-benzothieno-[2,3-d]-pyrimidin-2-yl)-phenyl]-essigsäure, Ethanolaminsatz. F. 119°;
2-[4-(4-(3,4-Methylendioxy-benzylamino)-5,6,7,8-tetrahydro-[1]-benzothieno-[2,3-d]-pyrimidin-2-yl)-phenyl]-essigsäure, F. 214.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g

NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 l isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 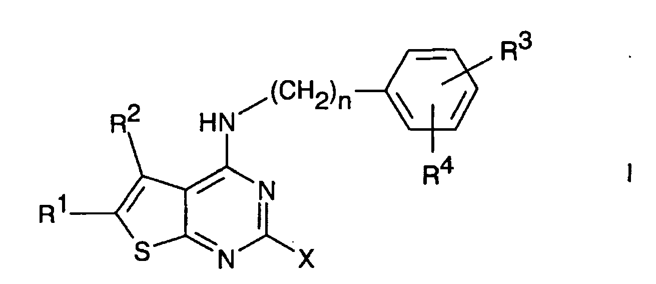 worin
R¹ und R² zusammen Alkylen mit 3-5 C-Atomen,
R³, R⁴ jeweils unabhängig voneinander H, A, OA oder Hal,
R³ und R⁴ zusammen auch Alkylen mit 3-5 C-Atomen, -O-CH₂-CH₂-, -O-CH₂-O- oder -O-CH₂-CH₂-O-,
X einfach durch R⁷ substituiertes R⁵ oder R⁶,
R⁵ lineares oder verzweigtes Alkylen mit 1-10 C-Atomen,
R⁶ Cycloalkylalkylen mit 6-12 C-Atomen,
R⁷ COOH, COOA, CONH₂, CONHA, CON(A)₂ oder CN,
A Alkyl mit 1 bis 6 C-Atomen,
Hal F, Cl, Br oder I,
m 1 oder 2
und
n 1, 2 oder 3
bedeuten,
sowie deren physiologisch unbedenklichen Salze.

2. Verbindungen der Formel I gemäß Anspruch 1
(a) 3-[4-(3-Chlor-4-methoxy-benzylamino)-5,6,7,8-tetrahydro-[1]-benzothieno-[2,3-d]-pyrimidin-2-yl]-propionsäure;
(b) 4-[4-(3,4-Methylendioxy-benzylamino)-5,6,7,8-tetrahydro-[1]-benzothieno-[2,3-d]-pyrimidin-2-yl]-buttersäure:
(c) 7-[4-(3,4-Methylendioxy-benzylamino)-5,6,7,8-tetrahydro-[1]-benzothieno-[2,3-d]-pyrimidin-2-yl]-heptansäure;
(d) 7-[4-(3-Chlor-4-methoxy-benzylamino)-5,6,7,8-tetrahydro-[1]-benzothieno-[2,3-d]-pyrimidin-2-yl]-heptansäure;
(e) 5-[4-(3-Chlor-4-methoxy-benzylamino)-5,6,7,8-tetrahydro-[1]-benzothieno-[2,3-d]-pyrimidin-2-yl]-valeriansäure;
(f) 2-{4-[4-(3-Chlor-4-methoxy-benzylamino)-5,6,7,8-tetrahydro-[1]-benzothieno-[2,3-d]-pyrimidin-2-yl]-cyclohexyl-1-yl}-essigsäure;
sowie deren physiologisch unbedenklichen Salze.

3. Verfahren zur Herstellung
von Verbindungen der Formel I nach Anspruch 1 sowie deren Salzen,
**dadurch gekennzeichnet, daß** man
a) eine Verbindung der Formel II 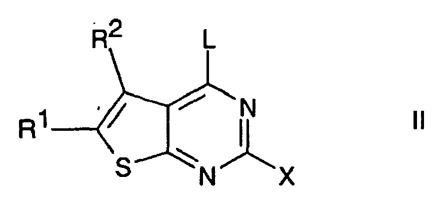 worin
R¹, R² und X die angegebenen Bedeutungen haben,
und L Cl, Br, OH, SCH₃ oder eine reaktionsfähige veresterte OH-Gruppe bedeutet,
mit einer Verbindung der Formel III 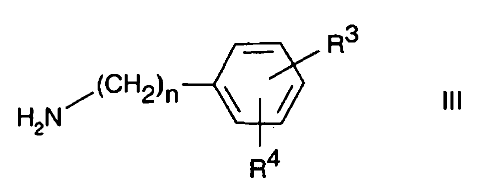 worin
R³, R⁴ und n die angegebenen Bedeutungen haben,
umsetzt,
oder
b) in einer Verbindung der Formel I einen Rest X in einen anderen Rest X umwandelt, indem man z.B. eine Estergruppe zu einer COOH-Gruppe hydrolysiert oder eine COOH-Gruppe in ein Amid oder in eine Cyangruppe umwandelt
und/oder daß man eine Verbindung der Formel I in eines ihrer Salze überführt.

4. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Krankheiten des Herz-Kreislaufsystems und zur Behandlung und/oder Therapie von Potenzstörungen.

7. Arzneimittel der Formel I nach Anspruch 1 und ihre physiologisch unbedenklichen Salze als Phosphodiesterase V-Hemmer.

8. Verwendung von Verbindungen der Formel I nach Anspruch 1 und/oder ihre physiologisch unbedenklichen Salze zur Herstellung eines Arzneimittels.

## Claims

1. Compounds of the formula I 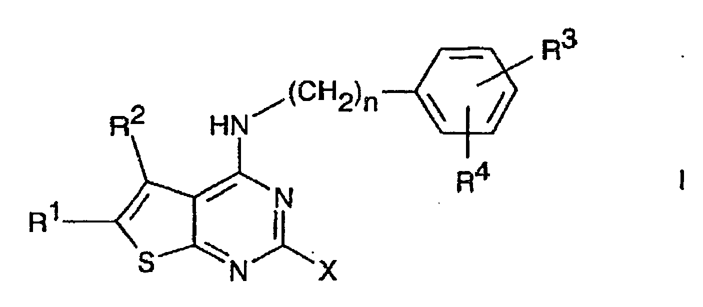 in which
R¹ and R² together denote alkylene having 3-5 C atoms,
R³, R⁴ each, independently of one another, denote H, A, OA or Hal,
R³ and R⁴ together also denote alkylene having 3-5 C atoms, -O-CH₂-CH₂-, -O-CH₂-O- or -O-CH₂-CH₂-O-,
X denotes mono-R⁷-substituted R⁵ or R⁶,
R⁵ denotes linear or branched alkylene having 1-10 C atoms,
R⁶ denotes cycloalkylalkylene having 6-12 C atoms,
R⁷ denotes COOH, COOA, CONH₂, CONHA, CON(A)₂ or CN,
A denotes alkyl having 1 to 6 C atoms,
Hal denotes F, Cl, Br or I,
m denotes 1 or 2
and
n denotes 1, 2 or 3,
and physiologically acceptable salts thereof.

2. Compounds of the formula I according to Claim 1
(a) 3-[4-(3-chloro-4-methoxybenzylamino)-5,6,7,8-tetrahydro[1]-benzothieno[2,3-d]pyrimidin-2-yl]propionic acid;
(b) 4-[4-(3,4-methylenedioxybenzylamino)-5,6,7,8-tetrahydro[1]-benzothieno[2,3-d]pyrimidin-2-yl]butyric acid;
(c) 7-[4-(3,4-methylenedioxybenzylamino)-5,6,7,8-tetrahydro[1]-benzothieno[2,3-d]pyrimidin-2-yl]heptanoic acid;
(d) 7-[4-(3-chloro-4-methoxybenzylamino)-5,6,7,8-tetrahydro[1]-benzothieno[2,3-d]pyrimidin-2-yl]heptanoic acid;
(e) 5-[4-(3-chloro-4-methoxybenzylamino)-5,6,7,8-tetrahydro[1]-benzothieno[2,3-d]pyrimidin-2-yl]valeric acid;
(f) 2-{4-(4-(3-chloro-4-methoxybenzylamino)-5,6,7,8-tetrahydro[1]-benzothieno[2,3-d]pyrimidin-2-yl)-cyclohex-1-yl}acetic acid;
and physiologically acceptable salts thereof.

3. Process for the preparation
of compounds of the formula I according to Claim 1, and salts thereof,
**characterised in that**
a) a compound of the formula II 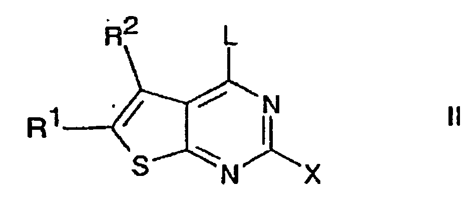 in which
R¹, R² and X have the meanings indicated,
and L denotes Cl, Br, OH, SCH₃ or a reactive esterified OH group,
is reacted with a compound of the formula III 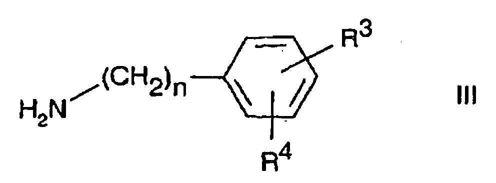 in which
R³, R⁴ and n have the meanings indicated,
or
b) a radical X in a compound of the formula I is converted into another radical X by, for example, hydrolysing an ester group to a COOH group or converting a COOH group into an amide or into a cyano group
and/or **in that** a compound of the formula I is converted into one of its salts.

4. Process for the preparation of pharmaceutical compositions, **characterised in that** a compound of the formula I according to Claim 1 and/or one of its physiologically acceptable salts is brought into a suitable dosage form together with at least one solid, liquid or semiliquid excipient or adjuvant.

5. Pharmaceutical composition, **characterised by** a content of at least one compound of the formula I according to Claim 1 and/or one of its physiologically acceptable salts.

6. Compounds of the formula I according to Claim 1 and physiologically acceptable salts thereof for combating diseases of the cardiovascular system and for the treatment and/or therapy of impotence.

7. Medicaments of the formula I according to Claim 1 and physiologically acceptable salts thereof as phosphodiesterase V inhibitors.

8. Use of compounds of the formula I according to Claim 1 and/or physiologically acceptable salts thereof for the preparation of a medicament.

## Revendications

1. Composés de formule I 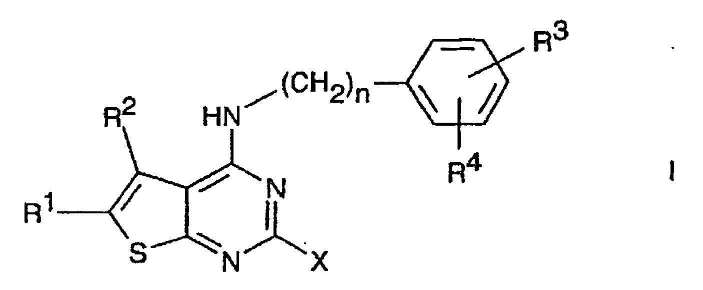 dans laquelle
R¹ et R² désignent ensemble alkylène ayant 3-5 atomes de C,
R³, R⁴ indépendamment l'un de l'autre, désignent chacun H, A, OA ou Hal,
R³ et R⁴ désignent également ensemble alkylène ayant 3-5 atomes de C, -O-CH₂-CH₂-, -O-CH₂-O- ou -O-CH₂-CH₂-O-,
X désigne R⁵ ou R⁶ monosubstitué par R⁷,
R⁵ désigne alkylène linéaire ou ramifié ayant 1-10 atomes de C,
R⁶ désigne cycloalkylalkylène ayant 6-12 atomes de C,
R⁷ désigne COOH, COOA, CONH₂, CONHA, CON(A)₂ ou CN,
A désigne alkyle ayant 1 à 6 atomes de C,
Hal désigne F, CI, Br ou I,
m vaut 1 ou 2
et
n vaut 1, 2 ou 3,
et les sels physiologiquement acceptables de ceux-ci.

2. Composés de formule I selon la revendication 1
(a) l'acide 3-[4-(3-chloro-4-méthoxybenzylamino)-5,6,7,8-tétrahydro[1]benzothiéno[2,3-d]pyrimidin-2-yl]propionique ;
(b) l'acide 4-[4-(3,4-méthylènedioxybenzylamino)-5,6,7,8-tétrahydro[1]benzothiéno[2,3-d]pyrimidin-2-yl]butyrique ;
(c) l'acide 7-[4-(3,4-méthylènedioxybenzylamino)-5,6,7,8-tétrahydro[1]benzothiéno[2,3-d]pyrimidin-2-yl]heptanoïque ;
(d) l'acide 7-[4-(3-chloro-4-méthoxybenzylamino)-5,6,7,8-tétrahydro[1]benzothiéno[2,3-d]pyrimidin-2-yl]heptanoïque ;
(e) l'acide 5-[4-(3-chloro-4-méthoxybenzylamino)-5,6,7,8-tétrahydro[1]benzothiéno[2,3-d]pyrimidin-2-yl]valérique ;
(f) l'acide 2-{4-(4-(3-chloro-4-méthoxybenzylamino)-5,6,7,8-tétrahydro[1]benzothiéno[2,3-d]pyrimidin-2-yl]-cyclohex-1-yl}acétique ;
et les sels physiologiquement acceptables de ceux-ci.

3. Procédé de préparation de composés de formule I selon la revendication 1, et de sels de ceux-ci,
**caractérisé en ce que**
a) un composé de formule II 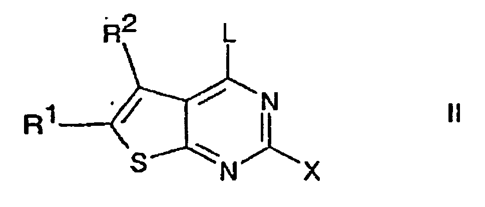 dans laquelle
R¹, R² et X ont les significations indiquées,
et L désigne CI, Br, OH, SCH₃ ou un groupement OH estérifié réactif,
est réagi avec un composé de formule III 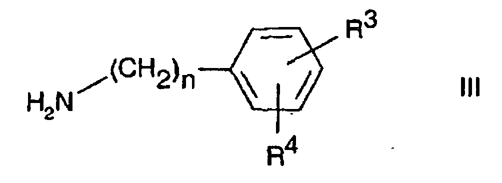 dans laquelle
R³, R⁴ et n ont les significations indiquées,
ou
b) un radical X dans un composé de formule I est converti en un autre radical X, par exemple par l'hydrolyse d'un groupement ester en un groupement COOH ou la conversion d'un groupement COOH en un amide ou en un groupement cyano
et/ou **en ce qu'**un composé de formule I est converti en l'un de ses sels.

4. Procédé de préparation de compositions pharmaceutiques, **caractérisé en ce qu'**un composé de formule I selon la revendication 1 et/ou l'un de ses sels physiologiquement acceptables est amené sous une forme de dosage convenable conjointement avec au moins un excipient ou adjuvant solide, liquide ou semi-liquide.

5. Composition pharmaceutique, **caractérisée par** une teneur en au moins un composé de formule I selon la revendication 1 et/ou un de ses sels physiologiquement acceptables.

6. Composés de formule I selon la revendication 1 et les sels physiologiquement acceptables de ceux-ci pour lutter contre les maladies du système cardiovasculaire et pour le traitement et/ou la thérapie de l'impotence.

7. Médicaments de formule I selon la revendication 1 et les sels physiologiquement acceptables de ceux-ci comme inhibiteurs de la phosphodiestérase V.

8. Utilisation de composés de formule I selon la revendication 1 et/ou de sels physiologiquement acceptables de ceux-ci pour la préparation d'un médicament.
